(19) Europäisches Patentamt
European Patent Office
Office européen des brevets

(11) Veröffentlichungsnummer : **0 460 714 B1**

# (12) EUROPÄISCHE PATENTSCHRIFT

(45) Veröffentlichungstag der Patentschrift : **08.12.93 Patentblatt 93/49**

(51) Int. Cl.$^5$ : **H01F 1/37**

(21) Anmeldenummer : **91113640.6**

(22) Anmeldetag : **11.06.87**

## (54) Verfahren zur Herstellung polarer und unpolarer superparamagnetischer Flüssigkeiten.

(30) Priorität : **12.06.86 DE 3619746**

(43) Veröffentlichungstag der Anmeldung : **11.12.91 Patentblatt 91/50**

(45) Bekanntmachung des Hinweises auf die Patenterteilung : **08.12.93 Patentblatt 93/49**

(84) Benannte Vertragsstaaten : **DE FR GB NL**

(56) Entgegenhaltungen :
**US-A- 3 531 413**
**US-A- 4 430 239**

(60) Veröffentlichungsnummer der früheren Anmeldung nach Art. 76 EPü : **0 249 229**

(73) Patentinhaber : **BASF Aktiengesellschaft**
**Carl-Bosch-Strasse 38**
**D-67063 Ludwigshafen (DE)**

(72) Erfinder : **Mair, Gunther, Dr.**
**Corneliusstrasse 15**
**W-6800 Mannheim 25 (DE)**
Erfinder : **Steck, Werner, Dr.**
**Auerstrasse 4**
**W-6700 Ludwigshafen (DE)**

**EP 0 460 714 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung unpolarer superparamagnetischer Flüssigkeiten aus mit anionischen Tensiden beschichteten superparamagnetischen Feststoffteilchen und unpolaren flüssigen Medien.

Außerdem betrifft die Erfindung ein neues Verfahren zur Herstellung polarer superparamagnetischer Flüssigkeiten aus mit sauren Phosphorsäureestern beschichteten superparamagnetischen Feststoffteilchen und polaren flüssigen Medien.

Unter Superparamagnetismus ist das ideale weichmagnetische Verhalten eines ferro- oder paramagnetischen Feststoffteilchens zu verstehen. Zu einem solchen Verhalten kommt es, wenn die magnetische Energie K x V eines Feststoffteilchens (K = Anisotropiekonstante, V = Teilchenvolumen) immer kleiner wird und irgendwann die Größenordnung der thermischen Energie k x T (k = Boltzmannsche Konstante, T = absolute Temperatur in Kelvin) erreicht, so daß kein permanenter Dipol mehr vorliegt. Für kubische Ferrite liegt der kritische größte Teilchendurchmesser, ab dem dieses Verhalten auftritt, bei ca. 5 bis 15 nm (vgl. C.P. Bean und J.D. Livingston, "Superparamagnetism", Journal of Applied Physics, Supplement to Volume 30, Number 4, Seiten 120S bis 129S, 1959). Dieser kritische Teilchendurchmesser entspricht im Falle der kubischen Ferrite - unter der Annahme, daß sie als monodisperse, weitgehend porenfreie kugelförmige Teilchen vorliegen - in etwa einer nach Brunauer, Emmet und Teller bestimmten BET-Oberfläche von 40 bis 130 $m^2/g$ (vgl. R. Brdicka, Grundlagen der Physikalischen Chemie, 7. Auflage, VEB-Verlag, Berlin, 1968, Seiten 546 bis 549).

Superparamagnetische Flüssigkeiten sind durch Tenside stabilisierte kolloidale Dispersionen superparamagnetischer Feststoffteilchen in polaren oder unpolaren flüssigen Medien. Im allgemeinen bedecken die Tenside die Oberfläche der superparamagnetischen Feststoffteilchen in Form einer überwiegend monomolekularen Schicht und verhindern so die Sedimentation der superparamagnetischen Feststoffteilchen in einem Schwerefeld, Magnetfeld und/oder in einem elektrischen Feld.

Da der Superparamagnetismus der ursprünglichen Feststoffteilchen weder durch die Tensidbeschichtung noch durch das kolloidale Dispergieren der beschichteten Feststoffteilchen in flüssigen Medien beeinträchtigt wird, zeigen die kolloidalen Dispersionen superparamagnetisches Verhalten. Mit anderen Worten, diese kolloidalen Dispersionen weisen insgesamt ein hysteresisfreies reversibles Magnetisierungs- und Demagnetisierungsverhalten auf.

Im Rahmen der vorliegenden Erfindung bezeichnet der Betriff "flüssiges Medium" die flüssigen molekulardispers miteinander vermischten Bestandteile einer kolloidalen Dispersion tensidbeschichteter superpramagnetischer Feststoffteilchen, wobei ein solches flüssiges Medium in der Hauptsache eine oder mehrere polare oder unpolare Flüssigkeiten wie Wasser, Kohlenwasserstoffe oder Öle enthält und darüber hinaus noch molekulardispers gelöste Zusatzstoffe wie Säuren, Basen, Salze, Gase oder Tenside enthalten kann. Dabei drückt der Begriff "polar" aus, daß die betreffenden Flüssigkeiten und flüssigen Medien in der Lage sind, miteinander oder mit Zusatzstoffen Dipol-Dipol-, Dipol-Ion und/oder Ion-Ion-Wechselwirkungen einzugehen, wogegen der Begriff "unpolar" darauf hindeutet, daß die betreffenden Flüssigkeiten und flüssigen Medien zu solchen Wechselwirkungen nicht in der Lage sind.

Im folgenden werden - der Kürze halber - superparamagnetische Flüssigkeiten auf der Basis unpolarer flüssiger Medien als "unpolare superparamagnetische Flüssigkeiten" und solche auf der Basis polarer flüssiger Medien als "polare superparamagnetische Flüssigkeiten" bezeichnet.

Im Rahmen der vorliegenden Erfindung werden agglomerierte sedimentierbare superparamagnetische Feststoffteilchen und sedimentierbare magnetische Feststoffteilchen unter dem Begriff "sedimentierbare Feststoffteilchen" zusammengefaßt.

Aus der US-A-3 843 540 ist ein Verfahren zur Herstellung superparamagnetischer Flüssigkeiten aus superparamagnetischen Feststoffteilchen und unpolaren flüssigen Medien bekannt, bei dem man die Teilchen aus wäßrigen Metallsalzlösungen, welche der chemischen Zusammensetzung der Teilchen entsprechen, mittels einer Base ausfällt, mit Tensiden beschichtet, in unpolare Flüssigkeiten überführt, aus diesen mittels Aceton ausflockt, abtrennt und mit Aceton wäscht, um sie anschließend in einer unpolaren Flüssigkeit zu redispergieren.

Das bekannte Verfahren umfaßt nicht das Ausflocken der Teilchen aus ihren Dispersionen in polaren oder unpolaren flüssigen Medien mittels Methanol oder das Waschen von Dispersionen auf der Basis unpolarer flüssiger Medien mit Basen und Wasser.

Aus der US-A-4 430 239 ist ein Verfahren zur Herstellung superparamagnetischer Flüssigkeiten aus superparamagnetischen Magnetitteilchen und polaren flüssigen Medien bekannt, bei dem man die Teilchen aus wäßrigen Metallsalzlösungen mittels einer Base ausfällt, in Wasser dispergiert, mit einem sauren Phosphorsäureester beschichtet, mit Aceton ausflockt, abtrennt und danach in einer polaren Flüssigkeit redispergiert.

Das bekannte Verfahren umfaßt nicht die Verwendung superparamagnetischer Feststoffteilchen und die

Verwendung einer polaren Flüssigkeit 1, welche niedriger siedet als der Hauptbestandteil des polaren flüssigen Mediums der superparamagnetischen Flüssigkeiten, die polare Flüssigkeit 2.

Die bekannten Verfahren weisen den Nachteil auf, daß sie unpolare oder polare superparamagnetische Flüssigkeiten mit einem nichtakzeptablen Anteil an sedimentierbaren Feststoffteilchen liefern, mit der Folge, daß die Sättigungsmagnetisierung $M_s$ der Flüssigkeiten niedriger ist, als man aufgrund der Ausgangsstoffmenge erwarten konnte. Außerdem weisen solche superparamagnetischen Flüssigkeiten eine unbefriedigende Stabilität auf, d.h., daß in ihnen im Lauf der Zeit irreversible Vorgänge ablaufen, die zu einer weiteren Bildung von sedimentierbaren Feststoffteilchen führen, wodurch die Anwendbarkeit solcher superparamagnetischer Flüssigkeiten erheblich eingeschränkt, wenn nicht sogar ganz aufgehoben wird. Zwar kann die Bildung sedimentierbarer Feststoffteilchen in einem geringen Umfang durch die Verwendung eines Überschusses an Tensiden, der weit über das hinausgeht, was für die Beschichtung der superparamagnetischen Feststoffteilchen notwendig ist, unterdrückt werden, dann aber erhöht sich bekanntermaßen (vgl. die US-A-3 843 540) die Viskosität der superparamagnetischen Flüssigkeiten in unerwünschter Weise.

Aufgabe der vorliegenden Erfindung war es, neue verbesserte Verfahren zur Herstellung polarer und unpolarer superparamagnetischer Flüssigkeiten zu finden.

Demgemäß wurde ein Verfahren zur Herstellung unpolarer superparamagnetischer Flüssigkeiten aus superparamagnetischen Feststoffteilchen und unpolaren flüssigen Medien gefunden, bei dem man die Teilchen aus wäßrigen Metallsalzlösungen, die der chemischen Zusammensetzung der Teilchen entsprechen, mittels einer Base ausfällt, mit anionischen Tensiden beschichtet und in eine unpolare Flüssigkeit überführt, wobei das Verfahren dadurch gekennzeichnet ist, daß man

a) die in dem wäßrigen Medium befindlichen beschichteten Teilchen mit Methanol ausflockt, abtrennt und in einer unpolaren Flüssigkeit redispergiert
und/oder

b) die bereits auf beliebige Weise in einem unpolaren flüssigen Medium dispergierten beschichteten Teilchen mit Methanol ausflockt, abtrennt und in einer unpolaren Flüssigkeit redispergiert.

In einer bevorzugten Ausführungsform des erfindungsgemäßen Verfahrens wird der Verfahrensschritt b zwei- oder mehrmals wiederholt.

In einer weiteren bevorzugten Ausführungsform werden die in erfindungsgemäßer Weise erhaltenen Dispersionen zunächst mit einer wäßrigen Base und danach solange mit Wasser gewaschen, bis das Waschwasser neutral reagiert und keine Anionen damit mehr nachweisbar sind.

Zudem wurde ein Verfahren zur Herstellung polarer superparamagnetischer Flüssigkeiten aus superparamagnetischen Feststoffteilchen und polaren flüssigen Medien gefunden, bei dem man die Teilchen aus wäßrigen Metallsalzlösungen, die der chemischen Zusammensetzung der Teilchen entsprechen, mittels einer Base ausfällt, mit sauren Phosphorsäureestern beschichtet und aus dem wäßrigen Medium in eine polare Flüssigkeit, die höher siedet als Wasser, überführt, wobei das Verfahren dadurch gekennzeichnet ist, daß man hierbei

a') die beschichteten superparamagnetischen Feststoffteilchen in eine zwischen 110 und 250°C siedende polare Flüssigkeit 1 überführt und daß man

b') die resultierende Dispersion nach dem Abtrennen des Wassers mit einer polaren Flüssigkeit 2 versetzt, welche höher siedet als die Flüssigkeit 1, wonach man die Flüssigkeit 1 aus der Dispersion entfernt.

Im folgenden werden diese Verfahren der Kürze halber zusammenfassend als "erfindungsgemäße Verfahren" bezeichnet.

Die erfindungsgemäßen Verfahren können auf alle para- oder ferromagnetische Feststoffe angewendet werden, sofern sie sich in wäßrigen Medien in kolloidal dispergierter Form in einer Teilchengröße von 5 bis 15 nm durch Ausfällen mittels Basen aus ihren wasserlöslichen Salzen herstellen lassen und nach ihrer Herstellung gegenüber den wäßrigen Medien stabil sind.

Beispiele für geeignete Feststoffe sind vor allem kubische Ferrite wie Magnetit, $\gamma$-Ferrit, Mangan-Eisen-Ferrit, Kobalt-Eisen-Ferrit, Nickel-Eisen-Ferrit, Zink-Eisen-Ferrit oder Mischkristalle aus Magnetit und $\gamma$-Ferrit (vgl. die US-A-3 531 413, US-A-4 430 239 und US-A-3 843 540).

Weitere Beispiele geeigneter Feststoffe oder Feststoffteilchen genügen der aus R.S. Tebble und D.J. Craik, "Magnetic Materials", Wiley-Interscience, London, Seiten 252 bis 270, 1969 (vgl. insbesondere Seite 256, Figur 7.2) bekannten Zusammensetzung der allgemeinen Formel II

$$M^1_w Zn_x Fe_2 O_4 \qquad II$$

mit den Variablen

| | |
|---|---|
| $M^1$ | Co, Ni oder Mn |
| w | 0,4 bis 0,5 |
| x | 0,5 bis 0,6 |
| w + x | 1 |

Die mit Hilfe der erfindungsgemäßen Verfahren hergestellten superparamagnetischen Flüssigkeiten enthalten die mit Tensiden beschichteten superparamagnetischen Feststoffteilchen kolloidal dispergiert in flüssigen Medien.

Bei diesen flüssigen Medien kann es sich sowohl um polare als auch um unpolare Medien handeln, wobei die polaren Medien in der Hauptsache polare Flüssigkeiten und die unpolaren Medien in der Hauptsache unpolare Flüssigkeiten enthalten.

Eine geeignete polare Flüssigkeit ist Wasser.

Beispiele geeigneter polarer Flüssigkeiten, welche höher sieden als Wasser, sind Ether wie Diethylenglykoldimethylether, Diethylenglykoldiethylether oder Diethylenglykolmonomethylether; $C_2$-$C_6$-Alkylester von $C_1$-$C_6$-Alkanmonocarbonsäuren, wie Essigsäurehexylester, Essigsäurecyclohexylester, Propionsäurepentylester, Buttersäureethylester, Pentancarbonsäuremethylester, Pentancarbonsäureethylester oder Hexancarbonsäuremethylester; oder $C_5$-$C_6$-Dialkylester von $C_1$-$C_7$-Alkandicarbonsäuren wie Malonsäuredihexylester, Bernsteinsäuredihexylester, Glutarsäuredipentylester, Adipinsäuredi-n-butylester, Pimelinsäuredi-n-butylester, Hexandicarbonsäuredi-n-propylester oder Azelainsäurediethylester (Heptandicarbonsäurediethylester). Polare Flüssigkeiten dieser Art sieden zwischen 110 und 250°C. Sie kommen als polare Flüssigkeiten 1 in Betracht. Von Vorteil ist der Essigsäurehexyl- und der Essigsäurecyclohexylester.

Beispiele weiterer geeigneter polarer Flüssigkeiten, welche höher sieden als die vorstehend genannten, sind höhere Dialkylester von Alkandicarbonsäuren wie Adipinsäuredinonylester, Adipinsäuredidecylester oder Adipinsäurediisodecylester, Hexandicarbonsäure-n-octylester, Azelainsäuredi(2-ethylhexyl)-ester oder Azelainsäuredi-n-octylester; höhere Dialkylester der Phthalsäure, Isophthalsäure oder Terephthalsäure wie Phthalsäuredinonylester, Phthalsäuredidecylester, Phthalsäurediisodecylester, Phthalsäureundecylester, Isophthalsäurediisodecylester oder Terephthalsäuredi-(2-ethylhexylester), höhere Trialkylester der Trimellithsäure wie Trimellithsäuretri-n-octylester oder Alkylbenzylester der Phthalsäure wie Phthalsäure-n-octyl-benzylester. Polare Flüssigkeiten dieser Art sieden im allgemeinen oberhalb 250°C. Sie kommen als polare Flüssigkeiten 2 in Betracht. Von Vorteil sind der Phthalsäurediisodecylester, der Adipinsäuredinonylester und der Adipinsäurediisodecylester.

Beispiele geeigneter unpolarer Flüssigkeiten sind aliphatische Kohlenwasserstoffe wie Hexane, Heptane, Octane, Nonane, Cyclopentan, Alkylcyclopentane, Cyclohexan, Alkylcyclohexane oder Petrolether eines Siedebereiches von 20 bis 160°C; aromatische Kohlenwasserstoffe wie Benzol oder Toluol; oder halogenierte aliphatische Kohlenwasserstoffe wie Methylenchlorid, Chloroform, Dichlordifluormethan oder die Dichlortetrafluorethane; von denen die Petrolether besonders vorteilhaft sind.

Weitere Beispiele geeigneter unpolarer Flüssigkeiten sind Mineralöle, Silikonöle, Öle auf der Basis von fluorierten Ethern, von fluorierten aliphatischen Kohlenwasserstoffen, von Alkylestern von Mono- und/oder Dicarbonsäuren, von hydrierten Poly-$\alpha$-olefinen, von Polyisobutylen, von Alkylaromaten, von hydrierten Poly-$\alpha$-olefinen und Alkylaromaten oder von Gemische aus solchen Ölen, wobei die Öle auf der Basis von hydrierten Poly-$\alpha$-olefinen und/oder von Alkylaromaten sowie Öle auf der Basis von Polyisobutylen und die aliphatischen Mineralöle von besonderem Vorteil sind.

Die Viskosität geeigneter Öle liegt im allgemeinen bei 2 bis 1000 mPas bei beispielsweise 20°C und/oder bei 1 bis 300 mPas bei beispielsweise 40°C. Im allgemeinen liegt ihre Verdampfungsrate bei 80°C unterhalb von $10^{-5}$, vorzugsweise $10^{-7}$ und insbesondere $10^{-8}$ $gcm^{-2}s^{-1}$. Sie weisen oftmals eine Dichte bei 20°C von 0,7 bis 0,9 $gcm^{-3}$ auf. Ihr Siedebereich liegt vorzugsweise bei 200 bis 600°C bei Normaldruck, bei 0,01 bis 0,3 mbar kann der Siedebereich auch bei 100 bis 300°C liegen.

Im Rahmen der erfindungsgemäßen Verfahren kommen für die Beschichtung der Feststoffteilchen vor allem wasserlösliche kationische und anionische Tenside oder elektrisch neutrale Tenside wie Ethylenoxid-Propylenoxid-Blockcopolymerisate in Frage; bevorzugt sind die anionischen Tenside.

Beispiele geeigneter anionischer Tenside sind demnach Alkylsulfonate und ihre Salze wie etwa Natriumeicosanylsulfonat oder Kaliumperfluoroctadecylsulfonat; Fettalkoholethersulfate und ihre Salze wie etwa Natrium-3,6-dioxaoctadecylsulfat; Fettalkoholphosphorsäuremonoester und ihre Mono- und Dialkalisalze wie etwa Natrium- und Dinatriumoctadecylphosphat; 2-Ethylhexylphosphat, Di-n-octylphosphat oder Oleylphosphat; Phosphorsäuremono- und -diester von alkoxylierten Alkoholen der allgemeinen Formel III

$$R^1\text{-O-}(R^2\text{-O})_p\text{-H} \qquad \text{III}$$

worin $R^1$ einen $C_6$-$C_{18}$-Alkylrest oder einen $C_{10}$-$C_{20}$-Arylalkylrest, $R^2$ einen $C_2$-$C_4$-Alkandiylrest und p eine Zahl von 1 bis 15 bezeichnet, wie Phosphorsäuremono- und -diester von ethoxyliertem n-Octylalkohol, ethoxyliertem Nonylalkohol, ethoxyliertem Decylalkohol, ethoxyliertem Pentadecylalkohol, propoxyliertem Octadecylalkohol, ethoxyliertem $\omega$-Phenylnonylalkohol, ethoxyliertem 2-Ethylhexanol, propoxyliertem 2-Ethylhexanol, ethoxyliertem 7-(p-Pentylphenyl)-heptylalkohol oder ethoxyliertem $\omega$-Phenyl-decylalkohol oder

Gemische aus solchen Estern; oder langkettige, einfach oder mehrfach olefinisch ungesättigte Fettsäuren mit 16 bis 25 Kohlenstoffatomen im Alkenylrest und ihre Alkali- oder Ammoniumsalze wie etwa Ölsäure oder Natrium- oder Ammoniumoleat.

Bevorzugt sind die Ölsäure und ihre Salze sowie die Phosphorsäuremono- und -diester von alkoxylierten Alkoholen der allgemeinen Formel III, worin $R^1$ einen $C_{12}$-$C_{17}$-Arylalkylrest, $R^2$ einen Ethylenrest und p eine Zahl von 1 bis 5 bezeichnet, wobei Natriumoleat und der Phosphorsäuremonoester von ethoxyliertem (p = 3 bis 5) ω-Phenylnonylalkohol ganz besonders bevorzugt sind.

Verfahrenstechnisch bieten die erfindungsgemäßen Verfahren keine Besonderheiten - d.h., daß zu ihrer Ausübung an und für sich keine speziell hierfür entwickelte und angepaßte Apparaturen notwendig sind und die einzelnen Verfahrensschritte - jeder für sich gesehen - auf bekannten chemischen Arbeitsmethoden beruhen.

Das erfindungsgemäße Verfahren zur Herstellung unpolarer superparamagnetischer Flüssigkeiten geht aus von der Herstellung kolloidaler wäßriger Dispersionen von mit anionischen Tensiden beschichteten superparamagnetischen Feststoffteilchen.

Im allgemeinen geschieht dies durch rasches Ausfällen der superparamagnetischen Feststoffteilchen unter Inertgas aus wäßrigen Lösungen entsprechend zusammengesetzter Salzgemische mittels in etwa stöchiometrischer Mengen an wäßrigen Basen wie etwa Natronlauge. Dabei kann die Teilchengröße der Feststoffteilchen durch Variation der Salz- und Basenkonzentration, der Fällungstemperatur- und -geschwindigkeit sowie des Verhältnisses q von Gehalt an zweiwertigem Eisen zu Gesamteisengehalt in den wäßrigen Lösungen vor dem Ausfällen in gewünschter Weise eingestellt werden. Üblicherweise wird in den Reaktionsgemischen aus Feststoffteilchen und wäßrigen Medien nach dem Ausfällen ein pH von 10 bis 12, vorzugsweise 11, eingestellt, wonach man die Reaktionsgemische eine gewisse Zeit, üblich sind 15 bis 60 Minuten, bei Raumtemperatur stehen läßt. Danach werden die Reaktionsgemische im allgemeinen neutralisiert. Die Feststoffteilchen werden nun in geeigneter Weise von den wäßrigen Medien abgetrennt, beispielsweise durch Sedimentation in einem Magnetfeld oder durch Filtration, und weitgehend elektrolytfrei gewaschen. Danach werden die Teilchen oder deren Aufschlämmungen in Wasser mit wäßrigen Lösungen eines oder mehrerer anionischer Tenside, beispielsweise mit wäßrigen Lösungen von Natriumoleat, versetzt, wobei man üblicherweise einen Überschuß an Tensiden verwendet, der über die Menge hinausgeht, wie sie normalerweise zur Bedeckung der superparamagnetischen Feststoffteilchen mit einer monomolekularen Schicht notwendig ist. Die so erhaltenen kolloidalen Dispersionen aus beschichteten Teilchen und wäßrigen Medien werden auf einen pH von 10 bis 12, vorzugsweise 11, eingestellt und längere Zeit, üblich sind 15 Minuten bis 3 Stunden, auf Temperaturen von 70 bis 100°C, vorzugsweise 90°C, unter Rühren erhitzt und gegebenenfalls anschließend neutralisiert.

Es ist üblich, diesen Verfahrensschritt insgesamt unter Ausschluß von Luftsauerstoff durchzuführen.

Die in den wäßrigen Medien befindlichen beschichteten Teilchen werden nun in dem erfindungsgemäßen Verfahrensschritt a durch Zugabe von Methanol ausgeflockt, wobei die Methanolvolumina den Volumina der wäßrigen Medien in etwa entsprechen. Von Vorteil ist es, wenn, bezogen auf die Volumina der wäßrigen Medien, ein Methanolüberschuß verwendet wird.

Die ausgeflockten beschichteten Teilchen werden von den polaren flüssigen Medien, die in der Hauptsache Wasser und Methanol enthalten, in bekannter Weise, z.B. durch Zentrifugieren und/oder durch Sedimentation in einem Magnetfeld, abgetrennt und gegebenenfalls mit frischem Methanol gewaschen und/oder getrocknet. Danach werden die Teilchen in den vorstehend genannten unpolaren Flüssigkeiten redispergiert, wobei es erfindungsgemäß von Vorteil ist, wenn man sie zunächst in unpolaren Flüssigkeiten wie Petrolether, welche niedriger sieden als die vorstehend genannten erfindungsgemäß anzuwendenden Öle, redispergiert.

Die in den unpolaren flüssigen Medien redispergierten beschichteten Teilchen werden nun in dem erfindungsgemäßen Verfahrensschritt b erneut mittels Methanol ausgeflockt, wobei die Methanolvolumina den Volumina der unpolaren flüssigen Medien in etwa entsprechen. Von Vorteil ist es, wenn, bezogen auf die Volumina der unpolaren flüssigen Medien, ein Methanolüberschuß verwendet wird.

Die ausgeflockten beschichteten Teilchen werden von den unpolaren flüssigen Medien abgetrennt, gegebenenfalls mit frischem Methanol gewaschen und/oder getrocknet und anschließend in unpolaren Flüssigkeiten redispergiert. Dabei ist es erfindungsgemäß von Vorteil, die ausgeflockten beschichteten Teilchen in den niedriger siedenden unpolaren Flüssigkeiten wie Petrolether zu redispergieren, wobei die Volumina der unpolaren Flüssigkeiten in etwa den Volumina der ursprünglichen unpolaren flüssigen Medien entsprechen.

Der erfindungsgemäße Verfahrensschritt b kann zwei- oder mehrmals durchgeführt werden.

Für den Fall, daß der erfindungsgemäße Verfahrensschritt a entfällt, erfolgt das Überführen der in den wäßrigen Medien befindlichen beschichteten Teilchen in die unpolaren flüssigen Medien bekanntermaßen durch Zugabe unpolarer Flüssigkeiten, Durchmischen der resultierenden polaren und unpolaren flüssigen Mischmedien und Phasentrennung, wonach man den erfindungsgemäßen Verfahrensschritt b obligatorisch zwei- oder mehrmals durchführt.

Die hiernach erhaltenen Dispersionen aus beschichteten Teilchen und unpolaren flüssigen Medien können zunächst mit einer wäßrigen Base wie Natronlauge, Kalilauge oder Ammoniakwasser und danach mit Wasser so lange gewaschen werden, bis das Waschwasser neutral reagiert und keine Anionen darin mehr nachweisbar sind, d.h., daß übliche und bekannte Nachweisreaktionen für die betreffenden Anionen negativ verlaufen.

Dabei kann sowohl die Wäsche mit Basen als auch die mit Wasser kontinuierlich in hierfür geeigneten Anlagen, beispielsweise in Extraktionskolonnen oder Gegenstromextraktoren, oder diskontinuierlich, beispielsweise in Scheidetrichtern oder Rührkesseln, durchgeführt werden. Es versteht sich von selbst, daß nach jedem Waschschritt eine Phasentrennung erfolgt. Wird das Waschen diskontinuierlich durchgeführt, dann ist es von Vorteil, jeweils mehrere kleine Portionen an wäßrigen Basen und Wasser anstelle jeweils einer großen Portion zu verwenden.

Im Rahmen des erfindungsgemäßen Verfahrens ist das Waschen mit wäßrigen Basen und mit Wasser dann obligatorisch, wenn die beiden Verfahrensschritte a und b nicht ausgeführt werden.

Die in dieser Weise erhaltenen superparamagnetischen Flüssigkeiten können, falls sich dies als notwendig erweist, noch in geeigneter Weise getrocknet werden. In Frage kommt hierbei beispielsweise die azeotrope Destillation des Wassers aus den unpolaren flüssigen Medien nach Zusatz von Verbindungen, die mit Wasser azeotrop siedende Gemische bilden.

Aus den nach dem erfindungsgemäßen Verfahren hergestellten Dispersionen aus beschichteten superparamagnetischen Feststoffteilchen und unpolaren flüssigen Medien können die Teilchen in andere unpolare flüssige Medien überführt werden. Dies kann z.B. dadurch geschehen, daß man in dem erfindungsgemäßen Verfahrensschritt b die ausgeflockten beschichteten Teilchen in einer anderen als der vor dem Ausflocken verwendeten unpolaren Flüssigkeit redispergiert. Dabei kann man die Menge der zweiten unpolaren Flüssigkeit so wählen, daß superparamagnetische Flüssigkeiten mit einem gewünschten Teilchengehalt resultieren. Bevorzugt wird jedoch die zweite unpolare Flüssigkeit zu der nach dem erfindungsgemäßen Verfahren erhaltenen superparamagnetischen Flüssigkeit hinzugegeben, wobei es von Vorteil ist, wenn die zweite unpolare Flüssigkeit sehr viel höher siedet als diejenige, welche die Basis des flüssigen unpolaren Mediums der superparamagnetischen Flüssigkeit bildet, und wobei ihre Menge so gewählt wird, daß die neue Dispersion nach dem Abdampfen der niedriger siedenden unpolaren Flüssigkeit aus dem Mischmedium den gewünschten Teilchengehalt aufweist. Als niedriger siedende unpolare Flüssigkeiten kommen hier vor allem Petrolether in Frage. Beispiele geeigneter höher siedender unpolarer Flüssigkeiten sind die vorstehend genannten Öle.

Bei dem erfindungsgemäßen Verfahren zur Herstellung polarer superparamagnetischer Flüssigkeiten erfolgt die Herstellung der wäßrigen Dispersionen von beschichteten Feststoffteilchen wie vorstehend bei dem Verfahren zur Herstellung unpolarer superparamagnetischer Flüssigkeiten beschrieben, nur das anstelle von beispielsweise Ölsäure saure Phosphorsäureester für die Beschichtung verwendet werden.

Beispiele für erfindungsgemäß geeignete saure Phosphorsäureester sind die vorstehend genannten Phosphorsäuremono- und -diester, von denen der Phosphorsäuremonoester des ethoxylierten (p = 3 bis 5) ω-Phenylnonylalkohols von besonderem Vorteil ist.

Abweichend von dem erfindungsgemäßen Verfahren zur Herstellung unpolarer superparamagnetischer Flüssigkeiten, werden nun zu den wäßrigen Dispersionen der mit sauren Phosphorsäureestern beschichteten Feststoffteilchen im Verfahrensschritt a' polare Flüssigkeiten 1 hinzugegeben, wobei deren Flüssigkeitsvolumina den Volumina der wäßrigen Medien in etwa entsprechen.

Beispiele für erfindungsgemäß geeignete polare Flüssigkeiten 1 sind die vorstehend genannten polaren Flüssigkeiten, welche zwischen 110 und 250°C sieden. Von diesen ist der Essigsäurehexyl- oder der Essigsäurecyclohexylester von ganz besonderem Vorteil.

Es ist indes auch möglich, zunächst die Feststoffteilchen herzustellen, zu isolieren und in einem Gemisch aus Wasser, sauren Phosphorsäureestern und polaren Flüssigkeiten 1 zu redispergieren.

Anschließend wird unter Rühren die Temperatur der Dispersionen auf 160 bis 180°C erhöht, und das darin enthaltene Wasser wird abdestilliert. Danach werden die wasserfreien Dispersionen während einer gewissen Zeit, beispielsweise während 30 Minuten, bei 160 bis 180°C nachgerührt.

Nach dem Abkühlen werden die Dispersionen aus den beschichteten Feststoffteilchen in den polaren Flüssigkeiten 1 im Verfahrensschritt b' mit der gewünschten Menge an polaren Flüssigkeiten 2 versetzt. Beispiele für erfindungsgemäß geeignete polare Flüssigkeiten 2 sind die vorstehend genannten polaren Flüssigkeiten, welche oberhalb 250°C sieden. Besonders vorteilhaft sind hierbei der Phthalsäurediisodecylester, der Adipinsäuredinonylester und der Adipinsäurediisodecylester. Hiernach werden die Flüssigkeiten 1 im Vakuum abdestilliert, und es resultieren polare superparamagnetische Flüssigkeiten.

Es ist von Vorteil, dieses Verfahren unter Inertgas auszuführen.

Darüber hinaus können die beiden erfindungsgemäßen Verfahren weitere Verfahrensschritte umfassen. So kann es sich als vorteilhaft erweisen, wenn man nach den erfindungsgemäßen Verfahrensschritten aus den dann jeweils vorliegenden Dispersionen aus beschichteten Teilchen und flüssigen Medien die sedimentierbaren Feststoffteilchen durch Zentrifugieren und/oder durch Sedimentieren in einem Magnetfeld abtrennt, wobei im allgemeinen dem Zentrifugieren den Vorzug gegeben wird.

Obwohl das Zentrifugieren im Rahmen der erfindungsgemäßen Verfahrens an sich nicht ausgeübt werden muß, wird es dennoch immer ausgeführt, da es sich hervorragend dazu eignet, die Vorteile, durch welche sich die erfindungsgemäßen Verfahren auszeichnen, zu belegen. Da beim Zentrifugieren den Dispersionen die sedimentierbaren Feststoffteilchen entzogen werden, erniedrigt sich die Sättigungsmagnetisierung $M_s$ von Dispersionen mit einem hohen Anteil an sedimentierbaren Feststoffteilchen gegenüber der Sättigungsmagnetisierung $M_s$ von vergleichbaren Dispersionen mit einem niedrigen Anteil an solchen Teilchen. Geht man also bei der Herstellung der betreffenden Dispersionen nach unterschiedlichen Verfahren vor, aber von den gleichen Ausgangsstoffen in den gleichen Mengen aus, so wird die unterschiedliche Sättigungsmagnetisierung $M_s$ der betreffenden Dispersionen, d.h. der superparamagnetischen Flüssigkeiten, zu einem direkten Maß des Erfolgs der unterschiedlichen Verfahren.

Die erfindungsgemäßen Verfahren weisen gegenüber dem Stand der Technik zahlreiche Vorteile auf. Mit ihrer Hilfe lassen sich unpolare und polare superparamagnetische Flüssigkeiten in einfacher und exakt reproduzierbarer Weise herstellen. Die so hergestellten superparamagnetischen Flüssigkeiten weisen eine deutlich höhere Sättigungsmagnetisierung $M_s$, eine höhere Stabilität und eine niedrigere Viskosität auf als diejenigen, welche nach dem Stand der Technik hergestellt worden sind.

Insgesamt sind die nach den erfindungsgemäßen Verfahren hergestellten polaren und unpolaren superparamagnetischen Flüssigkeiten hervorragend geeignet für Anwendungen auf den Gebieten der Wellenabdichtung, insbesondere in der Computer- und Vakuumtechnik; der Dämpfung von Lautsprechern und Schrittmotoren in der Elektrotechnik; der Dichtetrennung von Feststoffen wie Erzen oder Metallen, insbesondere im Bergbau oder der chemischen Industrie; der Mikroverkapselung von Wirkstoffen, insbesondere in der Medizin oder im Pflanzenschutz; der Ölpestbeseitigung; der Energiekonversion allgemein; der Flüssigkristallanzeigen; der magnetischen Ventile, insbesondere in der Hoch- und Ultrahochvakuumtechnik; oder der Treibstoffzusätze, insbesondere in der Weltraumtechnik; oder der Markierungstechnik, insbesondere in der Medizin, der Biochemie, der Mikrobiologie oder der Biotechnologie.

Beispiele

In den Beispielen und Vergleichsversuchen wurde die Sättigungsmagnetisierung $M_s$ ($nTm^3/g$) sowohl der superparamagnetischen Feststoffteilchen als auch der superparamagnetischen Flüssigkeiten in einem Magnetfeld der Stärke 160 kA/m direkt nach der Herstellung bestimmt. Im Falle superparamagnetischer Flüssigkeiten, welche man nach unterschiedlichen Verfahren, aber ausgehend von den gleichen Ausgangsstoffen in den gleichen Mengen hergestellt hatte und aus denen die sedimentierbaren Feststoffteilchen abzentrifugiert worden waren, war die Sättigungsmagnetisierung $M_s$ ein direktes Maß für den Erfolg der jeweils angewandten Herstellverfahren.

Daneben wurde noch in einigen Fällen die Ausbeute in % am tensidbeschichteten superparamagnetischen Feststoffteilchen über das Auswiegen der abzentrifugierten sedimentierbaren Feststoffteilchen bestimmt, wobei die theoretische Ausbeute zu 100 % angesetzt wurde.

Die innere Oberfläche ($m^2/g$) isolierter und getrockneter superparamagnetischer Feststoffteilchen wurde nach der BET-Methode gemessen. Die innere Oberfläche diente als Maß für die Teilchengröße.

Die Stabilität der polaren und unpolaren superparamagnetischen Flüssigkeiten wurde wie folgt ermittelt:

Man lagerte die superparamagnetischen Flüssigkeiten 7 Tage lang bei Raumtemperatur in senkrecht stehenden Röhrchen der Höhe 140 mm und des Durchmessers 3 mm. Nach dieser Zeit wurde mit Hilfe der Induktionsmethode geprüft, ob sich in den senkrecht stehenden Flüssigkeitssäulen ein Gradient der Sättigungsmagnetisierung $M_s$ gebildet hatte. Kam es nun zu einer Abnahme der Sättigungsmagnetisierung $M_s$ in den oberen Bereichen der Flüssigkeitssäulen und zu einer Zunahme der Sättigungsmagnetisierung $M_s$ in den unteren Bereichen, dann zeigte dies irreversible Veränderungen, begleitet von Sedimentation, in den betreffenden superparamagnetischen Flüssigkeiten an, d.h., die betreffenden superparamagnetischen Flüssigkeiten waren instabil. Das Ausmaß der Instabilität wurde anhand der nach der Induktionsmethode gemessenen Funktion "Sättigungsmagnetisierung $M_s$ = f (Höhe des Meßpunktes in der Flüssigkeitsäule)" beurteilt und mit:

1 keine Sedimentation - stabil;
2 geringfügige, gerade noch nachweisbare Sedimentation;
3 geringfügige, aber eindeutig nachweisbare Sedimentation;
4 starke Sedimentation;
5 praktisch vollständige Sedimentation;

benotet.

Die Viskosität (mPas) der superparamagnetischen Flüssigkeiten wurde mit Hilfe eines Rotationsviskosimeters bestimmt.

Beispiele 1a bis 1f

Herstellung unpolarer superparamagnetischer Flüssigkeiten nach dem entsprechenden erfindungsgemäßen Verfahren; Herstellvorschrift:

Es wurde eine Dispersion aus mit anionischen Tensiden beschichteten superparamagnetischen Magnetitteilchen und wäßrigem Medium aus 195 g $FeCl_3 \cdot 6H_2O$ und 103 g $FeCl_2 \cdot 4H_2O$ hergestellt. Hierzu wurden die Salze in 400 ml Wasser gelöst. Diese Lösung wurde unter Stickstoff zu 400 ml 8 n Natronlauge zugegeben. Das Reaktionsgemisch wurde unter Stickstoff durch Zugabe weiterer Natronlauge auf pH 11 gebracht, während 30 Minuten stehen gelassen und mittels Salzsäure neutralisiert. Die in dem Reaktionsgemisch vorliegenden Feststoffteilchen wurden unter Stickstoff in einem Magnetfeld sedimentiert, wonach man die wäßrigen Medien abdekantierte und die Feststoffteilchen unter Stickstoff so lange mit Wasser extrahierte, bis das Waschwasser neutral reagierte und keine Anionen darin mehr nachweisbar waren.

Daraus wurden die betreffenden Magnetitteilchen durch Zugabe von Methanol (1000 ml) ausgeflockt und wie beschrieben in Petrolether (1000 ml) redispergiert, erneut mit Methanol (1000 ml) ausgeflockt und anschließend wiederum in Petrolether (1000 ml) redispergiert.

Anschließend wurde die so erhaltene Dispersion zentrifugiert und in sechs gleiche Portionen aufgeteilt.

Zu diesen gab man jeweils eine auf einen theoretischen Feststoffgehalt der petroletherfreien superparamagnetischen Flüssigkeiten von 37,5 Gew.% bezogene Menge an unterschiedlichen höher siedenden unpolaren Flüssigkeiten (26,6 g), wobei die folgenden zur Anwendung kamen:

In Beispiel 1a:

Ein Polyisobutylenöl; physikalische Eigenschaften, Dichte (20°C): 0,83 $gcm^{-3}$, Viskosität (20°C): 118 mPas, Siedebereich (Normaldruck): 290 (5 % des Öls sind verdampft) - 390°C (95 % des Öls sind verdampft), massenmittleres Molgewicht: 320;

in Beispiel 1b:

Ein Alkylaromatenöl; physikalische Eigenschaften, Dichte (20°C): 0,87 $gcm^{-3}$, Viskosität (40°C): 38 mPas;

in Beispiel 1c:

Ein Alkylaromatenöl; physikalische Eigenschaften, Dichte (20°C): 0,86 $gcm^{-3}$, Viskosität (40°C): 37 mPas, Siedebereich (Normaldruck): 345 (5 % des Öls sind verdampft) - 385°C (95 % des Öls sind verdampft);

in Beispiel 1d:

Ein Monoalkylbenzolöl; physikalische Eigenschaften, Dichte (20°C): 0,83 $gcm^{-3}$, Siedepunkt von 145°C bei 0,2 mbar;

in Beispiel 1e:

Ein hydriertes Poly-$\alpha$-olefin-Öl; physikalische Eigenschaften, Dichte (20°C): 0,82 $gcm^{-3}$, Viskosität (40°C): 14 mPas;

in Beispiel 1f:

Ein Öl aus Alkylaromaten und hydrierten Poly-$\alpha$-olefinen; physikalische Eigenschaften: Dichte (20°C): 0,87 $gcm^{-3}$, Viskosität (40°C): 28 mPas.

Aus diesen so erhaltenen sechs Dispersionen wurde der Petrolether durch Abdampfen im Vakuum entfernt, wonach superparamagnetische Flüssigkeiten auf der Basis höher siedender unpolarer flüssiger Medien resultierten.

Diese wurden separat zentrifugiert, und anschließend wurde die Ausbeute an superparamagnetischen Magnetitteilchen insgesamt zu 82,6 % ermittelt.

Die Tabelle 1 faßt die ermittelten Werte für die Sättigungsmagnetisierung $M_s$ und die Stabilität der superparamagnetischen Flüssigkeiten zusammen. Die Werte der Beispiele 1a bis 1e aus Tabelle 1 können direkt mit den entsprechenden Werten der Vergleichsversuche Aa bis Ae aus Tabelle 2 wie folgt verglichen werden: 1a mit Aa, 1b mit Ab, 1c mit Ac, 1d mit Ad und 1e mit Ae.

Tabelle 1, Beisipele 1a bis 1f, Versuchsergebnisse

| Beispiel Nr. | Sättigungsmagnetisierung $M_S$ (nTm³/g) | Stabilität (Note) |
|---|---|---|
| 1a | 25 | 1 |
| 1b | 30 | 2 |
| 1c | 30 | 2 |
| 1d | 23 | 2 |
| 1e | 25 | 2 |
| 1f | 29 | 2 |

Vergleichsversuche Aa bis Ae

Herstellung superparamagnetischer Flüssigkeiten nach einem Verfahren des Standes der Technik; Herstellvorschrift:

Nach der bei den Beispielen 1a bis 1f angegebenen Herstellvorschrift wurde eine Dispersion aus mit anionischen Tensiden beschichteten superparamagnetischen Magnetitteilchen und wäßrigem Medium hergestellt.

Aus dieser Dispersion wurden die betreffenden Magnetitteilchen direkt in 1000 ml Petrolether überführt, indem man den Petrolether zur wäßrigen Dispersion hinzufügte, und das resultierende Gemisch aus zwei flüssigen Phasen kräftig durchmischte. Nach der Phasentrennung wurde das wäßrige Medium verworfen.

Die resultierende Dispersion aus beschichteten Magnetitteilchen und Petrolether wurde zentrifugiert und in fünf gleiche Portionen aufgeteilt. Diese fünf Portionen wurden mit jeweils 26,6 g jeweils eines der bei den Beispielen 1a bis 1e angegebenen Öle versetzt, wonach man den Petrolether daraus entfernte und die resultierenden superparamagnetischen Flüssigkeiten separat zentrifugierte.

Die Ausbeute an superparamagnetischen Magnetitteilchen insgesamt lag bei 72 %.

Die Tabelle 2 faßt die ermittelten Werte für die Sättigungsmagnetisierung $M_s$ und für die Stabilität der superparamagnetischen Flüssigkeiten zusammen. Die Werte der Vergleichsversuche Aa bis Ae aus Tabelle 2 können direkt mit den entsprechenden Werten der Beispiele 1a bis 1e aus Tabelle 2 wie folgt verglichen werden: Aa mit 1a, Ab mit 1b, Ac mit 1c, Ad mit 1d und Ae mit 1e.

Tabelle 2, Vergleichsversuche Aa bis Ae, Versuchsergebnisse

| Vgl. versuch | Sättigungsmagnetisierung $M_S$ (nTm³/g) | Stabilität (Note) |
|---|---|---|
| Aa | 21 | 2 |
| Ab | 12 | 3 |
| Ac | 11 | 3 |
| Ad | 4 | 4 |
| Ae | 3 | 4 |

Beispiele 2 bis 6

Herstellung unpolarer superparamagnetischer Flüssigkeiten nach dem entsprechenden erfindungsgemäßen Verfahren; Herstellvorschrift:

Nach der bei den Beispielen 1a bis 1f angegebenen Herstellvorschrift wurden fünf kolloidale Dispersionen aus mit anionischen Tensiden beschichteten superparamagnetischen Magnetitteilchen in wäßrigen Medien

hergestellt.

Aus diesen wäßrigen Dispersionen wurden die betreffenden Magnetitteilchen gemäß der in den Vergleichsversuchen Aa bis Af angegebenen Weise in jeweils 3000 ml Petrolether überführt.

Jede der nach der Phasentrennung resultierenden Dispersionen aus den betreffenden Magnetitteilchen und Petrolether wurde dreimal mit jeweils 1000 ml Natronlauge (pH 12) gewaschen.

Jede dieser Dispersionen wurde so lange mit 500 ml-Portionen an Wasser gewaschen, bis die Waschwasser neutral reagierten und mit Silbernitrat keine Chloridionen darin mehr nachweisbar waren.

Jede der fünf gewaschenen Dispersionen wurde zentrifugiert und anschließend mit jeweils 223,1 g jeweils eines Öls versetzt, wobei die angegebene Menge auf einen theoretischen Feststoffgehalt der superparamagnetischen Flüssigkeiten von 30 Gew.% bezogen war. Nach dem Entfernen des Petrolethers aus den kolloidalen Dispersionen resultierten fünf superparamagnetische Flüssigkeiten, die separat zentrifugiert wurden.

Folgende Öle kamen zur Anwendung:

In Beispiel 2:

Ein Polyisobutylenöl; physikalische Eigenschaften, Dichte (20°C): 0,83 $gcm^{-3}$, Viskosität (20°C): 118 mPas, Siedebereich (Normaldruck): 290 (5 % des Öls sind verdampft) - 390°C (95 % des Öls sind verdampft), massenmittleres Molgewicht: 320;

in Beispiel 3:

Ein Mineralöl (Hydrocracköl); physikalische Eigenschaften, Dichte (20°C): 0,8 $gcm^{-3}$, Viskosität (40°C): 18 mPas, Siedebereich (Normaldruck): 360 (5 % des Öls sind verdampft) - 520°C (95 % des Öls sind verdampft);

in Beispiel 4:

Ein hydriertes Poly-α-olefinen-Öl; physikalische Eigenschaften, Dichte (20°C): 0,82 $gcm^{-3}$, Viskosität (40°C): 14 mPas;

in Beispiel 5:

Ein Öl aus Alkylaromaten und hydrierten Poly-α-olefinen; physikalische Eigenschaften, Dichte (20°C): 0,87 $gcm^{-3}$, Viskosität (40°C): 28 mPas;

in Beispiel 6:

Ein Alkylaromatenöl; physikalische Eigenschaften, Dichte (20°C): 0,87 $gcm^{-3}$, Viskosität (40°C): 87 mPas, Siedebereich (Normaldruck): 360 (5 % des Öls sind verdampft) - 390°C (95 % des Öls sind verdampft).

Die Tabelle 3 faßt die ermittelten Werte für die Sättigungsmagnetisierung $M_s$, für die Stabilität und für die Viskosität der superparamagnetischen Flüssigkeiten zusammen.

Die Werte der Beispiele 2 bis 6 aus Tabelle 3 können direkt mit den Werten der Vergleichsversuche B bis F aus Tabelle 4 wie folgt verglichen werden: 2 mit B, 3 mit C, 4 mit D, 5 mit E und 6 mit F.

Tabelle 3, Beispiele 2 bis 6, Versuchsergebnisse

| Beispiel Nr. | Sättigungsmagnetisierung $M_S$ ($nTm^3/g$) | Viskosität (mPas) bei 20°C | 40°C | 80°C | Stabilität (Note) |
|---|---|---|---|---|---|
| 2 | 24 | 86 | 32 | 9 | 1 |
| 3 | 8 | 34 | 16 | 6 | 1 |
| 4 | 20 | 70 | 30 | 19 | 1 |
| 5 | 23 | 214 | 67 | 14 | 1 |
| 6 | 21 | 486 | 118 | 19 | 1 |

Vergleichsversuche B bis F

Herstellung unpolarer superparamagnetischer Flüssigkeiten nach einem Verfahren des Standes der Technik; Herstellvorschrift:

Gemäß der bei den Vergleichsversuchen Aa bis Ae angegebenen Herstellvorschrift wurden fünf Dispersionen von mit anionischen Tensiden beschichteten Magnetteilchen in Petrolether hergestellt.

Zu diesen fünf Dispersionen wurden direkt jeweils 223,1 g jeweils eines der bei den Beispielen 2 bis 6 aufgeführten Öle hinzugegeben, wonach man den Petrolether aus den Dispersionen entfernte.

Die resultierenden superparamagnetischen Flüssigkeiten wurden separat zentrifugiert.

Die Tabelle 4 faßt die ermittelten Werte für die Sättigungsmagnetisierung $M_s$, für die Stabilität und für die Viskosität der superparamagnetischen Flüssigkeiten zusammen. Die Werte der Vergleichsversuche B bis F aus Tabelle 4 können direkt mit den Werten der Beispiele 2 bis 6 aus Tabelle 3 wie folgt verglichen werden: B mit 2, C mit 3, D mit 4, E mit 5 und F mit 6.

Tabelle 4, Vergleichsversuche B bis F, Versuchsergebnisse

| Vgl. vers. | Sättigungsmagnetisierung $M_S$ ($nTm^3/g$) | Viskosität (mPas) bei 20°C | 40°C | 80°C | Stabilität (Note) |
|---|---|---|---|---|---|
| B | 18 | 56 | 21 | 6 | 3 |
| C | 3 | 47 | 21 | 7 | 5 |
| D | 10 | 45 | 20 | 7 | 4 |
| E | 15 | 119 | 38 | 9 | 4 |
| F | 12 | 251 | 26 | 13 | 4 |

Beispiel 7

Herstellung einer unpolaren superparamagnetischen Flüssigkeit nach dem entsprechenden erfindungsgemäßen Verfahren; Herstellvorschrift:

Nach der bei den Beispielen 1a bis 1f angegebenen Herstellvorschrift wurde eine Dispersion von mit anionischen Tensiden beschichteten superparamagnetischen Magnetitteilchen in Petrolether hergestellt.

Diese Dispersion wurde gemäß der bei den Beispiele 2 bis 6 angegebenen Herstellvorschrift dreimal mit 250 ml Natronlauge (pH 11) extrahiert und mit 250 ml-Portionen Wasser gewaschen, zentrifugiert und mit 213,5 g eines Polyisobutylenöls (zu den physikalischen Eigenschaften siehe das Beispiel 1a) versetzt, wobei diese Ölmenge auf einen theoretischen Feststoffgehalt der superparamagnetischen Flüssigkeit von 33 Gew.% bezogen war.

Die nach dem Entfernen des Petrolethers resultierende superparamagnetische Flüssigkeit wurde erneut zentrifugiert. Danach wurde ihre Sättigungsmagnetisierung $M_s$ zu 21 $nTm^3/g$ und ihre Viskosität bei 20°C zu 63, bei 40°C zu 24 und bei 80°C zu 7 mPas bestimmt. Die superparamagnetische Flüssigkeit war stabil (Note 1).

Vergleichsversuche G und H

Versuche zur Herstellung unpolarer superparamagnetischer Flüssigkeiten nach Ausflockungsmethoden des Standes der Technik; Vorschriften:

Bei dem Vergleichsversuch G wurde versucht, eine nach der bei den Beispielen 1a bis 1f angegebenen Versuchsvorschrift hergestellte und portionierte Dispersion aus mit anionischen Tensiden beschichteten Magnetitteilchen und wäßrigem Medium mit Flüssigkeiten des Standes der Technik wie Ethanol oder Aceton (vgl. die US-A-3 843 540) auszuflocken und weiterzuverarbeiten.

Bei dem Vergleichsversuch H wurde versucht, eine nach der bei den Vergleichsversuchen Aa bis Ae angegebenen Versuchsvorschrift hergestellte und portionierte Dispersion von mit anionischen Tensiden beschichteten superparamagnetischen Magnetitteilchen in Petrolether mit Flüssigkeiten des Standes der Technik wie Ethanol oder Acton (vgl. die US-A-3 843 540) auszuflocken und weiterzuverarbeiten.

In allen Fällen war aber das Redispergieren der ausgeflockten superparamagnetischen Magnetitteilchen in Petrolether nur noch in geringem Umfang möglich. Außerdem entstanden bei diesen Vorgehensweisen so viele sedimentierbare Feststoffteilchen, daß die Versuche abgebrochen werden mußten, weil die Ausbeute an superparamagnetischen Feststoffteilchen zu gering geworden war.

Beispiele 8 bis 12

Herstellung polarer superparamagnetischer Flüssigkeiten nach dem entsprechenden erfindungsgemäßen

Verfahren; Herstellvorschrift:

Für die Durchführung der Beispiele 8 bis 12 wurden aus jeweils 197,1 g $FeCl_3 \cdot 6\ H_2O$ und 103,4 g $FeCl_2 \cdot 2\ H_2O$ hergestellte, superparamagnetische Feststoffteilchen (Mischphase von $Fe_3O_4$ und $\gamma$-$Fe_2O_3$) verwendet.

Hierzu wurden die Metallchloride in der gewünschten Kombination mit dem gewünschten molaren Verhältnis in Wasser gelöst. Die Lösungen wurden unter Inertgas rasch zu vorgelegten in etwa stöchiometrischen Mengen an wäßriger Natronlauge hinzugegeben, wobei sich die Feststoffteilchen der gewünschten Zusammensetzung und Größe bildeten. Die Reaktionsgemische aus den Teilchen und den wäßrigen Medien wurden durch Zugabe von Salzsäure auf pH 11 gebracht, wonach man die Reaktionsgemische 30 Minuten bei Raumtemperatur stehen ließ. Danach wurden die Reaktionsgemische neutralisiert. Die darin befindlichen Feststoffteilchen wurden vom wäßrigen Medium durch Filtration abgetrennt, mehrmals mit Wasser gewaschen und an der Luft bei 120°C getrocknet.

Jeder der 5 Proben wurde unter Stickstoff in einer Mischung aus 200 ml Wasser, 20 oder 30 g des Phosphorsäuremonoesters des ethoxylierten (p = 3 bis 5) ω-Phenylnonylalkohols und 400 ml Essigsäurecyclohexylester redispergiert und unter Rühren auf 160°C erhitzt, wobei das Wasser abdestillierte.

Die resultierenden 5 Dispersionen aus Feststoffteilchen und der polaren Flüssigkeit 1 wurden während 30 Minuten bei 160°C nachgerührt. Nach dem Abkühlen wurden sie zentrifugiert, um vorhandene sedimentierbare Feststoffteilchen abzutrennen und um die Ausbeute an Feststoffteilchen in diesen Dispersionen zu bestimmen.

Anschließend gab man soviele Gewichtsteile an polaren Flüssigkeiten 2 hinzu, wie es dem gewünschten Gehalt an Feststoffteilchen in den polaren superparamagnetischen Flüssigkeiten entsprach und destillierte die polare Flüssigkeit 1 im Vakuum ab.

Die Tabelle 5 gibt Auskunft über die Art und Menge der verwendeten Stoffe, die Ausbeute an Feststoffteilchen und über die an den polaren superparamagnetischen Flüssigkeiten ermittelten Meßwerte.

Tabelle 5

Beispiele 8 bis 12: Stoffe und Versuchsergebnisse

| Beispiel Nr. | saurer Phosphorsäureester (g) | Ausbeute an Feststoffteilchen in der polaren Flüssigkeit 1 (%) | polare superparamagnetische Flüssigkeit: Gew.% Feststoffteilchen I | polare Flüssigkeit 2 | Sättigungsmagnetisierung ($nTm^3/g$) | Viskosität bei 20°C (mPas) | Stabilität (Note) |
|---|---|---|---|---|---|---|---|
| 8 | 20 | 66 | 37,5 | Phthalsäurediisodecylester | 23 | - | 1 |
| 9 | 30 | 33 | 37,5 | Adipinsäuredinonylester | 16 | 57 | 1 |
| 10 | 30 | 35 | 37,5 | Adipinsäurediisodecylester | 17,7 | 75 | 1 |
| 11 | 30 | 34 | 44,4 | Adipinsäurediisodecylester | 20 | 99 | 2 |
| 12 | 30 | 36 | 37,5 | Phthalsäurediisodecylester | 19,6 | 348 | 1-2 |

Vergleichsversuch I

Herstellung einer polaren superparamagnetischen Flüssigkeit nach einem Verfahren des Standes der Technik (US-PS 4 430 239); Herstellvorschrift:

Nach der in Beispiel 1 der US-PS 4 430 239 angegebenen Vorschrift wurden ca. 100 g an superparamagnetischem Magnetit hergestellt und in 313 g des sauren Phosphorsäuremonoesters des ethoxylierten (p = 3 bis 5) ω-Phenylnonylalkohols und 3130 ml Wasser dispergiert.

Zu dieser Dispersion wurden 3000 ml Aceton zugegeben, wodurch die beschichteten superparamagnetischen Magnetitteilchen ausgeflockt wurden. Die ausgeflockten Teilchen wurden von der Wasser/Aceton-Mischung abgetrennt und mit 6000 ml Aceton gewaschen.

Die gewaschenen, feuchten Teilchen wurden in 250 ml Azelainsäuredi-(2-ethylhexyl)ester redispergiert, wonach man restliches Wasser und Aceton abdestillierte.

Es resultierte eine polare superparamagnetische Flüssigkeit mit einem Gehalt an superparamagnetischen Feststoffteilchen von 33 Gew.%. Sie wies eine Sättigungsmagnetisierung von 39 $nTm^3/g$ und eine Viskosität bei 20°C von 140 mPas auf. Sie enthielt aber noch größere Mengen an sedimentierbaren Feststoffteilchen. Diese wurden abzentrifugiert, wodurch der Anteil an superparamagnetischen Feststoffteilchen in der polaren superparamagnetischen Flüssigkeit auf 23,5 Gew.% sank. Aber selbst diese geringer konzentrierte superparamagnetische Flüssigkeit war nicht stabil, sondern zeigte eine merkliche Sedimentation (Note 3 bis 4).

**Patentansprüche**

1. Verfahren zur Herstellung unpolarer superparamagnetischer Flüssigkeiten aus superparamagnetischen Feststoffteilchen und unpolaren flüssigen Medien durch Ausfällung der Teilchen aus wäßrigen Metallsalzlösungen, welche der chemischen Zusammensetzung der Teilchen entsprechen, mittels einer Base, Beschichtung der Teilchen mit einem anionischen Tensid und Überführung der Teilchen in eine unpolare Flüssigkeit, dadurch gekennzeichnet, daß man

   a) die in dem wäßrigen Medium befindlichen beschichteten Teilchen mit Methanol ausflockt, abtrennt und in einer unpolaren Flüssigkeit redispergiert

   und/oder

   b) die bereits auf beliebige Weise in einem unpolaren flüssigen Medium dispergierten beschichteten Teilchen mit Methanol ausflockt, abtrennt und in einer unpolaren Flüssigkeit redispergiert.

2. Das Verfahren nach Anspruch 1, dadurch gekennzeichnet, daß man die Variante b zwei- oder mehrmals wiederholt.

3. Das Verfahren nach Anspruch 1 oder 2, dadurch gekennzeichnet, daß man die hiernach erhaltenen Dispersionen zunächst mit einer wäßrigen Base und danach solange mit Wasser wäscht, bis das Waschwasser neutral reagiert und keine Anionen mehr darin nachweisbar sind.

4. Verfahren zur Herstellung unpolarer superparamagnetischer Flüssigkeiten aus superparamagnetischen Feststoffteilchen und unpolaren flüssigen Medien durch Ausfällung der Teilchen aus wäßrigen Metallsalzlösungen, welche der chemischen Zusammensetzung der Teilchen entsprechen, mittels einer Base, Beschichtung der Teilchen mit einem anionischen Tensid und Überführung der Teilchen in eine unpolare Flüssigkeit, dadurch gekennzeichnet, daß man die hiernach erhaltenen Dispersionen zunächst mit einer wäßrigen Base und danach solange mit Wasser wäscht, bis das Waschwasser neutral reagiert und keine Anionen mehr darin nachweisbar sind.

5. Verfahren zur Herstellung polarer superparamagnetischer Flüssigkeiten aus superparamagnetischen Feststoffteilchen und polaren flüssigen Medien durch Ausfällen der Teilchen aus wäßrigen Metallsalzlösungen, welche der chemischen Zusammensetzung der Teilchen entsprechen, mittels einer Base, Beschichtung der Teilchen mit einem sauren Phosphorsäureester und Überführung der Teilchen aus dem wäßrigen Medium in eine polare Flüssigkeit, welche höher siedet als Wasser, dadurch gekennzeichnet, daß man hierbei

   a') die beschichteten superparamagnetischen Feststoffteilchen in eine zwischen 110 und 250°C siedende polare Flüssigkeit 1 überführt und daß man

   b') die resultierende Dispersion nach dem Abdestillieren des Wassers mit einer polaren Flüssigkeit 2 versetzt, welche höher siedet als die Flüssigkeit 1, wonach man die Flüssigkeit 1 aus der Dispersion

entfernt.

## Claims

1. A process for the preparation of a nonpolar superparamagnetic liquid from superparamagnetic solid particles and a nonpolar liquid medium by precipitating the particles from an aqueous metal salt solution which corresponds to the chemical composition of the particles by means of a base, coating the particles with an anionic surfactant and transferring the particles to a nonpolar liquid, wherein
   a) the coated particles present in the aqueous medium are flocculated with methanol, separated off and redispersed in a nonpolar liquid
   or
   b) the coated particles already dispersed in any manner in a nonpolar liquid medium are flocculated with methanol, separated and redispersed in a nonpolar liquid.

2. A process as claimed in claim 1, wherein variant b is repeated two or more times.

3. A process as claimed in claim 1 or 2, wherein the dispersion obtained thereby is washed first with an aqueous base and then with water until the washwater is neutral and anions are no longer detected therein.

4. A process for the preparation of a nonpolar superparamagnetic liquid from superparamagnetic solid particles and a nonpolar liquid medium by precipitating the particles from an aqueous metal salt solution which corresponds to the chemical composition of the particles by means of a base, coating the particles with an anionic surfactant and transferring the particles to a nonpolar liquid, wherein the dispersion obtained thereby is washed first with an aqueous base and then with water until the washwater is neutral and anions are no longer detectable therein.

5. A process for the preparation of a polar superparamagnetic liquid from superparamagnetic solid particles and a polar liquid medium by precipitating the particles from an aqueous metal salt solution which corresponds to the chemical composition of the particles by means of a base, coating the particles with an acidic phosphoric ester and transferring the particles from the aqueous medium to a polar liquid which has a boiling point higher than that of water, wherein
   a') the coated superparamagnetic solid particles are transferred to a polar liquid 1 which boils at from 110 and 250°C and
   b') a polar liquid 2 which has a boiling point higher than that of the liquid 1 is added to the resulting dispersion after the water has been distilled off, and the liquid 1 is then removed from the dispersion.

## Revendications

1. Procédé de préparation de liquides super-paramagnétiques non polaires à partir de particules solides super-paramagnétiques et de milieux liquides non polaires par précipitation des particules à partir de solutions aqueuses de sels métalliques correspondant à la composition chimique des particules à l'aide d'une base, revêtement des particules par un agent tensioactif anionique et transfert des particules dans un liquide non polaire, caractérisé en ce que :
   a) on flocule les particules revêtues qui se trouvent dans le milieu aqueux à l'aide du méthanol, on les sépare et on les redisperse dans un liquide non polaire, et/ou
   b) on flocule à l'aide du méthanol les particules revêtues déjà dispersées de manière quelconque dans un milieu liquide non polaire, on les sépare et on les redisperse dans un liquide non polaire.

2. Procédé selon la revendication 1, caractérisé en ce que l'on répète deux ou plusieurs fois la variante opératoire b).

3. Procédé selon la revendication 1 ou 2, caractérisé en ce qu'on lave ensuite les dispersions obtenues, d'abord à l'aide d'une base aqueuse puis à l'eau jusqu'à ce que les eaux de lavage aient une réaction neutre et qu'on n'y trouve plus d'anions.

4. Procédé de préparation de liquides super-paramagnétiques non polaires à partir de particules de matières solides super-paramagnétiques et de milieux liquides non polaires par précipitation des particules à partir

de solutions aqueuses de sels métalliques correspondant à la compostion chimique des particules à l'aide d'une base, revêtement des particules par un agent tensioactif non-ionique et transfert des particules dans un liquide non polaire, caractérisé en ce qu'on lave ensuite les dispersions obtenues, d'abord avec une base aqueuse puis avec de l'eau, jusqu'à réaction neutre des eaux de lavage et absence d'anions dans ces eaux.

**5.** Procédé de préparation de liquides super-paramégnétiques polaires à partir de particules de matières solides super-paramagnétiques et de milieux liquides polaires par précipitation des particules à partir de solutions aqueuses de sels métalliques correspondant à la composition chimique des particules à l'aide d'une base, revêtement des particules par un ester phosphorique acide et transfert des particules du milieu aqueux dans un liquide polaire bouillant plus haut que l'eau, caractérisé en ce que :

a') on transfère les particules de matières solides super-paramagnétiques revêtues dans un liquide polaire 1 bouillant de 110 à 250 degrés C, et

b') on ajoute à la dispersion obtenue, après distillation de l'eau, un liquide polaire 2 bouillant plus haut que le liquide 1, puis on élimine le liquide 1 de la dispersion.